# FASCICULE DE BREVET EUROPEEN

(11) **EP 3 681 589 B1**
(45) Date de publication et mention de la délivrance du brevet: **25.11.2020**
(21) Numéro de dépôt: 19744687.5
(22) Date de dépôt: 24.07.2019
(51) Int. Cl.: A61N 1/05, A61N 1/375, A61N 1/372, A61B 5/00

(54) **CAPSULE CARDIAQUE AUTONOME IMPLANTABLE À TÊTE ORIENTABLE ET LIMITEUR DE COUPLE**
IMPLANTIERBARE AUTONOME HERZKAPSEL MIT AUSRICHTBAREM KOPF UND DREHMOMENTBEGRENZER
IMPLANTABLE AUTONOMOUS HEART CAPSULE WITH ORIENTABLE HEAD AND TORQUE LIMITER

(30) Priorité: 25.09.2018 FR 1871085
(43) Date de publication de la demande: 22.07.2020
(73) Titulaire: Cairdac, 92160 Antony (FR)
(72) Inventeur: REGNIER, Willy, 91160 Longjumeau (FR); NGUYEN-DINH, An, 37520 La Riche (FR)
(74) Mandataire: Dupuis-Latour, Dominique
(86) Numéro de dépôt international: PCT/EP2019/069893
(87) Numéro de publication internationale: WO 2020/064172

(56) Documents cités:
- US-A1- 2012 172 892
- US-A1- 2014 378 991
- US-A1- 2015 374 976
- US-B2- 9 974 948

## Description

L'invention concerne les dispositifs médicaux implantables, notamment les dispositifs de type capsule autonome implantable.

L'invention concerne plus particulièrement ceux de ces dispositifs qui se présentent sous forme d'une capsule autonome implantée dans une cavité cardiaque (ventricule, oreillette ou même cavité cardiaque gauche artérielle), ci-après désignée "capsule autonome", "capsule *leadless"* ou simplement "capsule" (le caractère autonome de la capsule n'étant pas en soi une caractéristique nécessaire de l'invention). Ces capsules autonomes sont dépourvues de toute liaison physique à un dispositif principal implanté (tel qu'un boitier de générateur d'impulsions de stimulation) ou non implanté (périphérique externe tel que programmateur ou dispositif de monitoring pour le suivi à distance du patient), et sont dénommées pour cette raison *"leadless",* pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde.

L'invention n'est toutefois pas limitée à un type particulier de capsule, ni même d'implant *leadless,* et elle est applicable indifféremment à de nombreux autres types de dispositifs médicaux implantables, quelle que soit leur destination fonctionnelle, cardiaque ou autre, par exemple à des capsules destinées à diffuser *in situ* un agent pharmacologique actif.

Dans ce cas d'une application cardiaque, la capsule surveille en continu le rythme du patient et délivre si nécessaire au cœur des impulsions électriques de stimulation, de resynchronisation et/ou de défibrillation en cas de troubles du rythme détecté par la capsule. La capsule peut être une capsule épicardique, fixée à la paroi extérieure du cœur, ou bien une capsule endocavitaire, fixée à la paroi intérieure d'une cavité ventriculaire ou auriculaire, ou bien encore une capsule fixée sur une paroi d'un vaisseau à proximité du myocarde.

La capsule comprend divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Les US 2009/0171408 A1 (Solem), US 2015/374976 A1 (Regnier), US 2017/0151429 A1 (Regnier) et WO 2018/122244 A1 (Regnier) décrivent divers exemples de telles capsules *leadless* intracardiaques. L'invention concerne plus particulièrement les problèmes liés à l'implantation *in situ* de telles capsules lorsque celles-ci sont pourvues à leur extrémité distale d'un organe d'ancrage apte à pénétrer dans les tissus d'une paroi corporelle au site d'implantation choisi.

Un exemple typique d'un tel organe d'ancrage comprend une vis hélicoïdale saillante prolongeant axialement le corps de la capsule et destinée à pénétrer dans le tissu cardiaque par vissage au site d'implantation. Ce mode d'ancrage n'est toutefois pas limitatif de l'invention, qui peut s'appliquer également à d'autres types d'organes d'ancrage, par exemple mettant en œuvre des aiguilles, des crochets, des barbes, etc. pénétrant dans les tissus pour y assujettir de façon permanente le dispositif médical.

Dans le cas des capsules endocavitaires (c'est-à-dire des capsules à fixer à la paroi intérieure d'une cavité ventriculaire ou auriculaire, par opposition aux capsules épicardiques, fixées à la paroi extérieure du cœur), la "délivrance", c'est-à-dire à la mise en place au site d'implantation, consiste à monter la capsule à l'extrémité du cathéter-guide d'un accessoire d'implantation, puis à la faire progresser le long du réseau veineux périphérique et la diriger jusqu'au site choisi, par exemple l'apex de la cavité ventriculaire droite. Une fois le site d'implantation atteint, le praticien imprime à la capsule par l'intermédiaire du cathéter-guide des mouvements combinés de translation axiale (pour faire progresser la capsule vers l'avant puis exercer une pression contre la paroi cardiaque) et de rotation de la capsule sur elle-même (pour opérer le vissage de l'organe d'ancrage dans l'épaisseur de la paroi cardiaque). Une fois la capsule fermement ancrée dans la paroi cardiaque, l'opérateur procède au "largage" de la capsule, c'est-à-dire à sa désolidarisation d'avec l'accessoire d'implantation, la capsule devenant alors totalement autonome.

L'une des difficultés est, au stade de la fixation de la capsule dans la paroi, d'éviter tout risque de carottage des tissus du fait d'un vissage excessif. Pour cela, il est impératif, au moment du vissage de l'organe d'ancrage, de ne pas dépasser un couple limite (ci-après "couple de carottage") au-delà duquel la vis d'ancrage risquerait de déchirer localement les tissus sous l'effet de la rotation de la vis sans avance de celle-ci, jusqu'à provoquer une lacération des tissus et, à l'extrême, une perforation de la paroi avec risque de tamponnade (en particulier dans le cas d'une implantation dans une paroi mince telle que le septum interauriculaire ou la zone apicale du ventricule droit).

Les WO 2017/202724 A1 (Ollivier), US 2014/378991 A1 (Ollivier) et US 2014/378992 A1 ou US 9 974 948 B2 (Ollivier) proposent une solution mettant en œuvre un ensemble spécifique combinant capsule et accessoire d'implantation. Plus précisément, l'extrémité distale du cathéter-guide de l'accessoire d'implantation, d'une part, et la région proximale de la capsule (celle opposée à l'organe d'ancrage), d'autre part, sont pourvues de moyens coopérants de solidarisation en translation et en rotation, découplables pour larguer la capsule une fois en place, pourvus d'un mécanisme débrayable simple permettant lors du vissage de l'organe d'ancrage de limiter le couple appliqué à la capsule par l'outil d'implantation.

Ce mécanisme est constitué par un ressort hélicoïdal utilisé en compression radiale (c'est-à-dire pour son effet de striction), disposé à l'extrémité du cathéter-guide et enroulé autour d'une tige d'arrimage de la capsule en partie distale de celle-ci. Le ressort joue alors un double rôle de moyen de liaison débrayable et de limiteur de couple à l'encontre d'une action de vissage excessive qui pourrait entrainer un carottage des tissus. Le praticien peut ainsi poursuivre sans risque la rotation du cathéter-guide, car le couple supplémentaire apparaissant du fait de la réaction de la vis ancrée dans les tissus sera absorbé par la liaison entre le ressort et la tige d'arrimage de la capsule en partie distale.

Cette solution, si elle est efficace, n'en présente pas moins l'inconvénient de nécessiter un accessoire d'implantation spécifiquement adapté à la capsule, car la fonction de limitation du couple est localisée à l'interface capsule/accessoire d'implantation. Elle ne peut donc pas être mise en œuvre avec les équipements d'implantation standard dont disposent les praticiens, ce qui en limite l'acceptation par ces derniers.

Un inconvénient supplémentaire de cette technique connue est la nécessité de prévoir en partie distale de la capsule (c'est-à-dire du côté de l'extrémité libre, opposée à la vis d'ancrage), une tige d'arrimage suffisamment longue pour qu'elle puisse être emprisonnée par le ressort de l'outil d'implantation. Cette exigence augmente nécessairement la longueur hors-tout de la capsule, ce qui va à l'encontre des exigences de miniaturisation extrême imposées aux capsules *leadless.*

L'un des buts de l'invention est de proposer une solution à cette difficulté, grâce à un système de limitation de couple qui soit intrinsèque à la capsule, permettant ainsi d'assurer la délivrance de cette dernière indépendamment de l'accessoire d'implantation utilisé par le praticien.

Un autre but encore de l'invention est de proposer une telle capsule dont la position en rotation du corps par rapport à la paroi puisse être éventuellement modifiée après implantation, c'est-à-dire une capsule ménageant une possibilité de rotation du corps cylindrique autour de son axe sans entrainer pour autant de mouvement de vissage/dévissage de l'organe d'ancrage.

Un autre but encore de l'invention est de proposer une telle capsule dont le coût de fabrication soit réduit, notamment grâce à un nombre réduit de pièces et l'utilisation de technologies et de composants éprouvés dans des applications similaires.

Plus précisément, l'invention propose une capsule autonome implantable comprenant, de manière en elle-même connue notamment d'après le US 2015/374976 A1 précité, un corps tubulaire logeant un ensemble d'éléments fonctionnels de la capsule et, à une extrémité proximale de la capsule, un ensemble frontal comprenant un organe d'ancrage pour l'ancrage de la capsule à une paroi d'un organe du patient,

De façon caractéristique de l'invention, l'ensemble frontal est, avec l'organe d'ancrage, mobile en rotation relative axiale par rapport au corps tubulaire, et la capsule comprend en outre, entre l'ensemble frontal et le corps tubulaire, un organe de couplage débrayable agencé pour autoriser ladite rotation relative lorsque le corps tubulaire reçoit une sollicitation externe en rotation qui lui est appliquée avec l'organe d'ancrage ancré dans la paroi de l'organe du patient, l'organe d'ancrage exerçant alors un couple de réaction supérieur à un couple seuil prédéterminé, et empêcher ladite rotation relative en l'absence de sollicitation externe en rotation appliquée au corps tubulaire.

Dans un premier mode de réalisation de l'invention, l'organe de couplage débrayable comprend au moins une interface de friction entre l'ensemble frontal et le corps tubulaire, et un élément élastiquement déformable agencé pour appliquer un effort de compression axiale entre une première face d'appui solidaire en rotation du corps tubulaire et une seconde face d'appui solidaire en rotation de l'ensemble frontal.

De préférence, en l'absence de sollicitation externe en rotation appliquée au corps tubulaire, l'élément élastiquement déformable applique à l'endroit de l'interface de friction un effort supérieur au couple seuil prédéterminé.

De préférence également, l'organe de couplage débrayable comprend une bague support solidaire en rotation de l'organe d'ancrage et venant en appui frottant contre une surface annulaire frontale du corps tubulaire située à l'extrémité proximale de celui-ci.

Dans ce dernier cas, l'élément élastiquement déformable peut appliquer l'effort de compression axiale contre la bague support en direction de la surface annulaire frontale, et la bague support peut comprendre deux faces, comprenant une première face tournée vers la surface annulaire frontale, avec un degré de liberté en rotation et un premier contact frottant à l'interface entre la première face et la surface annulaire frontale, et une seconde face opposée tournée vers l'élément élastiquement déformable, avec un degré de liberté en rotation et un second contact frottant à l'interface entre la seconde face et l'élément élastiquement déformable.

Dans ce premier mode de réalisation, le corps tubulaire et l'ensemble frontal ne sont pas mobiles entre eux en translation axiale.

Dans un deuxième mode de réalisation de l'invention, l'organe de couplage débrayable comprend une bague support solidaire en rotation de l'organe d'ancrage, une surface frontale du corps tubulaire, située à l'extrémité proximale de celui-ci en vis-à-vis de la bague support, et des formes conjuguées prévues respectivement sur la bague support et sur la surface frontale, les formes conjuguées permettant un emboitement mutuel du corps tubulaire avec l'ensemble frontal pour permettre un entrainement en rotation de la bague support, et corrélativement de l'organe d'ancrage solidaire de celle-ci, par l'organe d'emboitement de la surface frontale sous l'effet de la sollicitation externe en rotation appliquée au corps tubulaire.

Dans ce deuxième mode de réalisation, le corps tubulaire et l'ensemble frontal peuvent être mobiles entre eux en translation axiale, et le corps tubulaire porter à son extrémité proximale un élément destiné à venir en appui contre la paroi de l'organe du patient, cet élément étant apte à exercer durant la pénétration de l'organe d'ancrage un effort de réaction axial propre à déboiter axialement les formes conjuguées respectives de la bague support et de la surface frontale.

Avantageusement, l'organe de couplage débrayable comprend un élément élastiquement déformable agencé pour solliciter axialement les formes conjuguées en rapprochement mutuel, notamment un élément élastiquement déformable agencé pour subir, sous l'effet dudit effort de réaction axial, une déformation d'un état non déformé à un état de déformation maximale correspondant audit couple seuil prédéterminé ; cet élément élastiquement déformable peut en particulier être un élément du groupe constitué par : bague ondulée, ressort de compression, lame ressort, bague plastique déformable et entretoise en matériau souple.

Dans un troisième mode de réalisation de l'invention, le corps tubulaire et l'ensemble frontal ne sont pas mobiles entre eux en translation axiale, et l'organe de couplage débrayable comprend un élément élastiquement déformable agencé pour subir, sous l'effet dudit couple de réaction, une déformation entre :un état où l'élément élastiquement déformable exerce entre les formes conjuguées un effort de striction radiale assurant l'emboitement mutuel du corps tubulaire avec l'ensemble frontal, et un état de déformation maximale propre à déboiter le corps tubulaire et l'ensemble frontal, pour un couple de réaction correspondant audit couple seuil prédéterminé.

On va maintenant décrire divers exemples de réalisation de la présente invention, en référence aux dessins annexés où les mêmes références désignent d'une figure à l'autre des éléments identiques ou fonctionnellement semblables.
La Figure 1 illustre des dispositifs médicaux de type capsule *leadless* dans leur environnement, avec divers exemples de sites d'implantation dans, sur ou à proximité du cœur d'un patient.
La Figure 2 est une coupe longitudinale en vue perspective d'un exemple de capsule *leadless,* montrant la configuration mécanique des différents éléments situés à l'intérieur de l'enveloppe tubulaire de la capsule.
La Figure 3 est une vue perspective générale de l'ensemble frontal selon l'invention, situé à l'extrémité proximale d'une capsule telle que celle de la Figure 2.
La Figure 4 est une vue en coupe, en perspective, d'un premier mode de réalisation d'un ensemble frontal d'une capsule selon l'invention.
La Figure 5 est une vue éclatée, en perspective, des différents éléments constituant l'ensemble de la Figure 4.
La Figure 6 est une vue en coupe d'un deuxième mode de réalisation d'un ensemble frontal d'une capsule selon l'invention.
La Figure 7 est une vue en coupe d'une variante du mode de réalisation illustré Figure 6.
La Figure 8 montre, isolément, le capot métallique frontal de la capsule et la bague support conjuguée solidaire de la vis d'ancrage, pour le mode de réalisation de la Figure 6.
La Figure 9 illustre une première variante des deux éléments de la Figure 8.
La Figure 10 illustre une seconde variante des deux éléments de la Figure 8.
La Figure 11 illustre un troisième mode de réalisation d'un ensemble frontal d'une capsule selon l'invention.
La Figure 12 illustre deux éléments conjugués utilisés par l'ensemble frontal de la Figure 11.

On va maintenant décrire plusieurs exemples de réalisation d'une capsule selon l'invention.

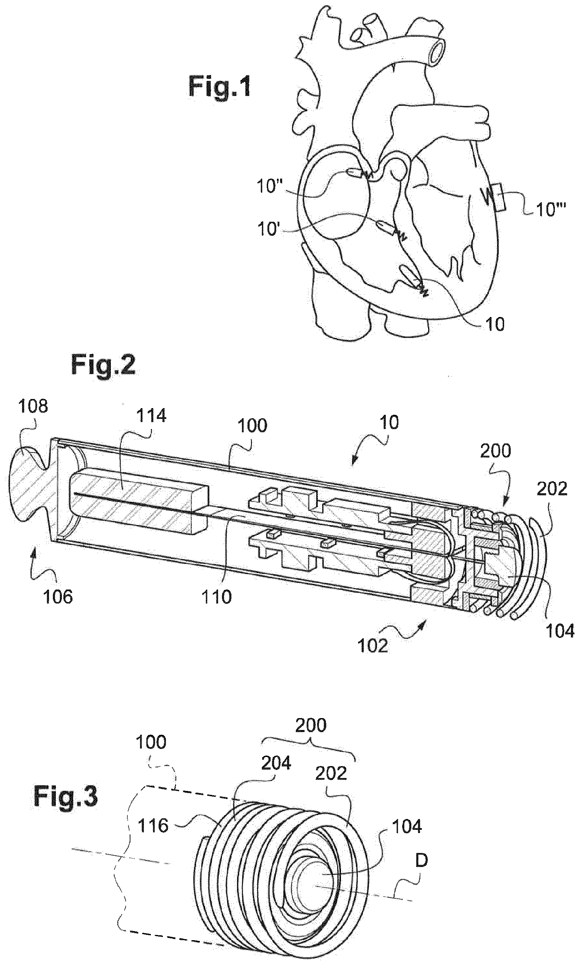

Sur la Figure 1, on a représenté diverses possibilités de sites d'implantation d'un dispositif de type *leadless,* dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme en 10". Le dispositif peut également être une capsule épicardique placée sur une région externe du myocarde, comme en 10"'. Dans chaque cas, la capsule *leadless* est fixée à la paroi cardiaque au moyen d'un système d'ancrage saillant tel qu'une vis hélicoïdale pénétrant dans le tissu cardiaque pour le maintien sur le site d'implantation. D'autres systèmes d'ancrage sont utilisables, et ne modifient en aucune façon la mise en œuvre de la présente invention.

La Figure 2 est une coupe longitudinale en vue perspective d'un exemple de capsule *leadless* montrant la configuration mécanique des différents éléments situés à l'intérieur de l'enveloppe tubulaire de la capsule.

La capsule *leadless* 10 se présente sous forme extérieure d'un implant avec un corps comprenant un corps tubulaire allongé cylindrique 100 enfermant les divers circuits électroniques et d'alimentation de la capsule ainsi que, dans l'exemple (non limitatif) illustré, un récupérateur d'énergie à ensemble pendulaire. Les dimensions typiques d'une telle capsule sont un diamètre de l'ordre de 6 mm pour une longueur d'environ 25 à 40 mm.

Le corps tubulaire allongé 100 est fermé à son extrémité avant (proximale) 102 par un ensemble frontal 200 portant une vis hélicoïdale 202 pour l'ancrage de la capsule sur une paroi d'une cavité cardiaque, comme illustré plus haut à propos de la Figure 1 (ce mode d'ancrage n'étant bien entendu aucunement limitatif). Une électrode de détection/ stimulation 104, en contact avec le tissu cardiaque au site d'implantation, assure le recueil des potentiels de dépolarisation cardiaque et/ou l'application d'impulsions de stimulation.

L'extrémité arrière (distale) opposée 106 du corps tubulaire 100 de la capsule 10 présente une forme arrondie atraumatique, et elle est pourvue de moyens appropriés 108 tels qu'une forme de préhension pour la liaison à un cathéter-guide ou autre accessoire d'implantation utilisable au moment de la mise en place ou de l'explantation de la capsule. Ces moyens permettent, par une action contrôlée par le praticien de rotation et de translation combinées du cathéter-guide, de guider la capsule vers le site d'implantation puis d'y assujettir celle-ci par vissage de l'élément d'ancrage dans la paroi.

La capsule 10 est avantageusement pourvue d'un module de récupération d'énergie comprenant un ensemble pendulaire inertiel qui, à l'intérieur de la capsule, oscille au gré des diverses sollicitations externes auxquelles la capsule est soumise, et qui peuvent notamment résulter des mouvements de la paroi à laquelle est ancrée la capsule, qui sont transmis à l'implant par la vis d'ancrage 202, et/ou des variations du débit du flux sanguin dans le milieu environnant l'implant, qui produisent des oscillations de l'implant au rythme des battements cardiaques, et/ou des vibrations diverses transmises par les tissus cardiaques.

L'ensemble pendulaire peut notamment être constitué d'une lame piézoélectrique 110 encastrée en 112 à l'une de ses extrémités et dont l'extrémité opposée, libre, est couplée à une masse inertielle mobile 114. La lame piézoélectrique 110 est une lame flexible élastiquement déformable qui constitue, avec la masse inertielle 114, un système pendulaire de type masse-ressort qui, du fait de l'inertie de la masse inertielle 114, soumet la lame 110 à une déformation de type vibratoire de part et d'autre d'une position neutre ou non déformée, correspondant à sa position stable de repos en l'absence de toute sollicitation. La lame piézoélectrique 110 assure en outre une fonction de transducteur mécanoélectrique permettant de convertir en charges électriques la sollicitation mécanique qui lui est appliquée lorsqu'elle est fléchie, charges récupérées par des électrodes (non représentées) formées à la surface de la lame pour produire un signal électrique qui, après redressement, stabilisation et filtrage alimentera les divers circuits électroniques de la capsule.

La Figure 3 illustre, isolément, l'ensemble frontal 200 de la capsule monté à l'extrémité proximale du corps tubulaire 100 de la capsule, avec l'électrode 104 destinée à venir en appui contre la surface du tissu à l'endroit du site d'implantation et la vis hélicoïdale 202 destinée à pénétrer dans ce tissu pour permettre l'ancrage de la capsule au site d'implantation.

La vis 202 est solidaire en rotation d'une bague support 204, la vis étant par exemple soudée à cette bague support, les deux éléments étant métalliques, par exemple en acier inoxydable tel qu'un acier 316 L ou encore un métal tel que le titane, le tantale ou un alliage nickel-titane de type nitinol.

(On notera incidemment que tous les éléments solidaires du corps tubulaire 100 seront désignés par une référence numérique de type 1*nn,* tandis que tous ceux qui sont solidaires de l'ensemble frontal 200 seront désignés par une référence numérique de type 2*nn*)*.*

Le corps tubulaire 100 est terminé en vis-à-vis de la bague support 204 par un capot de fermeture 116 de forme sensiblement plate et contre lequel la bague support 204 vient en appui. Le capot de fermeture 116, tout comme le corps tubulaire 100 de la capsule, sont des éléments métalliques, par exemple en titane ou en acier inoxydable tel qu'un acier 316 L, ou encore en un alliage nickel-titane de type nitinol, les deux pièces 100 et 116 étant par exemple soudées ensemble.

De façon caractéristique de l'invention, l'ensemble frontal 200, comprenant donc la vis d'ancrage 202 avec sa bague support 204, dispose par rapport au corps tubulaire 100 et à son capot de fermeture 116 d'un degré de liberté en rotation axiale (c'est-à-dire autour de l'axe central D).

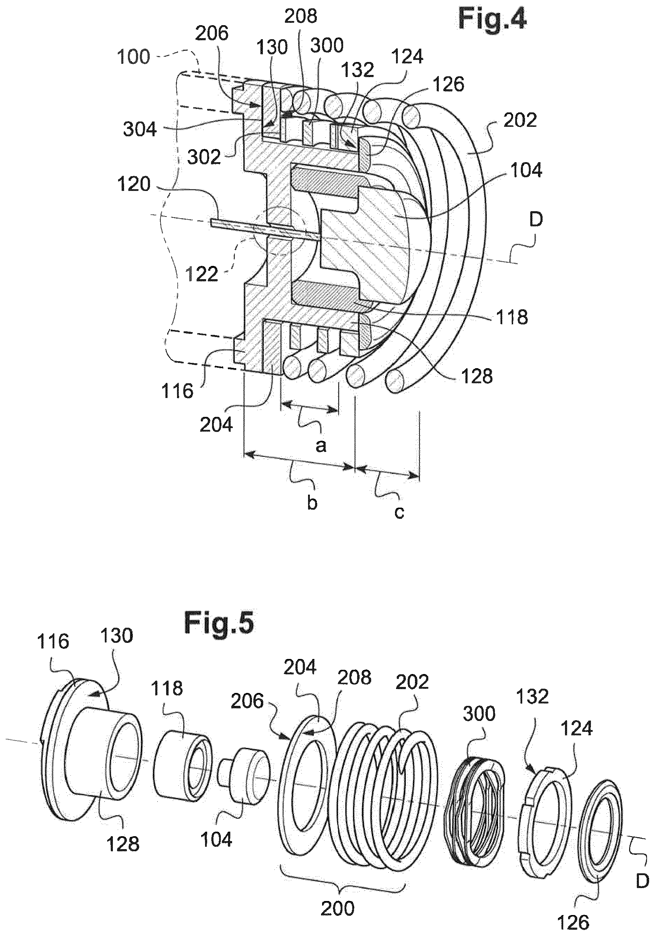

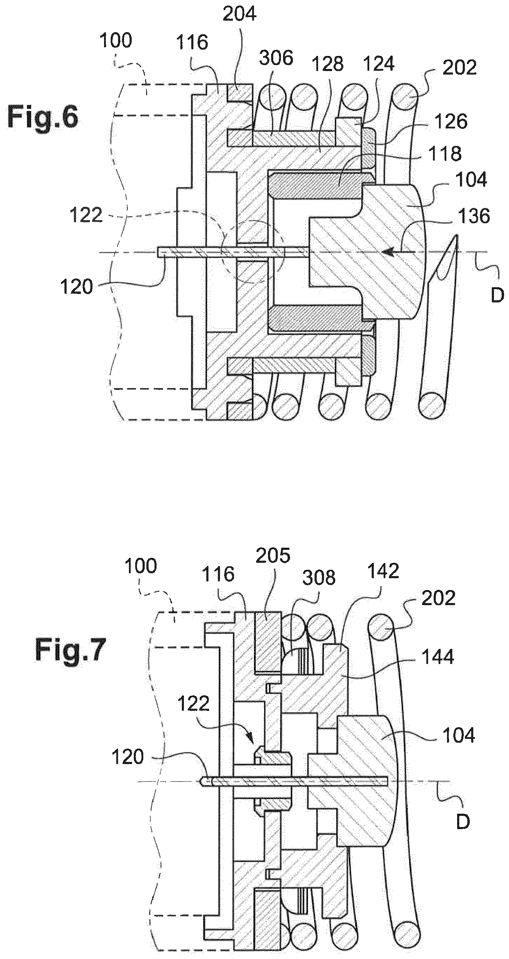

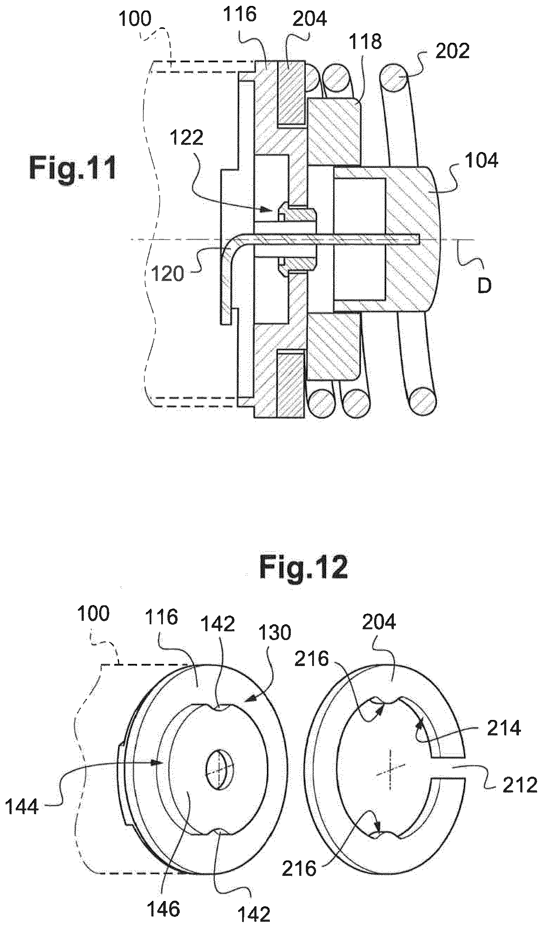

Dans certaines configurations particulières, telles que celles des modes de réalisation des Figures 6 et 7 que l'on décrira plus loin, l'ensemble frontal 200 et le corps tubulaire 100 peuvent également présenter un degré de liberté en translation axiale (c'est-à-dire parallèlement à l'axe D). En revanche, dans d'autres modes de réalisation, tels que ceux illustrés Figures 4 et 11, l'ensemble frontal 200 ne dispose que d'un seul degré de liberté en rotation axiale par rapport au corps tubulaire 100 et aux éléments solidaires de ce dernier.

De façon également caractéristique de l'invention, le corps tubulaire 100 ainsi que les éléments dont il est solidaire, d'une part, et l'ensemble frontal 200, d'autre part, sont couplés entre eux par des moyens débrayables, grâce à divers mécanismes dont on décrira plusieurs réalisations en référence aux Figures 4 à 12. Ces moyens débrayables sont en particulier agencés pour assurer une fonction de limiteur de couple entre le corps tubulaire (qui reçoit une sollicitation externe au moment de l'implantation via l'outil de pose manipulé par le praticien) et la vis d'ancrage (qui pénètre dans le tissu, et pour lequel il est indispensable d'éviter tout effet de carottage).

Le mécanisme limiteur de couple permet, lorsque le corps tubulaire est sollicité en rotation et applique donc à la vis d'ancrage un couple correspondant qui permet la pénétration de celle-ci dans le tissu, d'assurer la transmission de ce couple d'entrainement tant qu'il est inférieur à un couple seuil prédéterminé (choisi pour être inférieur au couple de carottage) et, si le couple d'entrainement dépasse ce seuil, empêche le carottage, soit en limitant ce couple (qui continuera à être transmis du corps tubulaire à l'ensemble frontal), soit en débrayant totalement l'ensemble frontal du corps tubulaire (mettant ainsi fin à la transmission du couple d'entrainement). Le couple seuil prédéterminé déclenchant le découplage de l'ensemble frontal d'avec le corps tubulaire est typiquement inférieur à 1N.cm, pour éviter tout risque de carottage par la vis hélicoïdale au moment de l'implantation. Concrètement, le couple de réaction augmente de façon relativement brusque lorsque la face avant de la capsule (en l'occurrence, l'électrode 104) touche la surface du tissu cardiaque, exerçant alors sur la vis d'ancrage une force de réaction axiale qui, en l'absence d'action de débrayage ou de limitation du couple, serait susceptible de produire un carottage.

On verra également que les divers mécanismes de couplage débrayable décrits permettent, une fois le débrayage opéré, de conserver un degré de liberté en rotation du corps tubulaire par rapport à l'ensemble frontal, de manière à pouvoir modifier éventuellement l'orientation du corps tubulaire *in situ* sans pour autant modifier la position de la vis hélicoïdale, donc en maintenant l'ancrage tel qu'il avait été initialement assuré.

Cette modification ultérieure de l'orientation angulaire résultera d'une action de rotation exercée sur le corps tubulaire, étant entendu que, en l'absence d'une telle sollicitation, l'orientation de la capsule ne sera pas modifiée et restera celle qui avait été initialement obtenue.

### Premier mode de réalisation de l'invention

En référence aux Figures 4 et 5, on va décrire un premier mode de réalisation de l'invention, dans lequel l'ensemble frontal 200 et le corps tubulaire 100 sont mobiles entre eux suivant un unique degré de liberté, en rotation autour de l'axe D.

Le corps tubulaire 100 et son capot de fermeture 116 forment un ensemble solidaire avec l'électrode 104 portée par une bague support d'électrode 118 en matériau électriquement isolant, par exemple un polymère thermoplastique de polyuréthane de type *Tecothane*® ou un polymère de type PET (poly(téréphtalate d'éthylène)) ou PEEK (polyétheréthercétone), ou autre matière plastique injectable. L'électrode 104 est reliée aux circuits électroniques situés à l'intérieur du corps tubulaire 100 par un fil de liaison 120 traversant le capot 116 et isolé de celui-ci par une traversée 122 (illustrée plus en détail sur les Figures 7 et 11).

Le capot 116 comporte une excroissance tubulaire 128 recevant et supportant un capuchon 124 en forme de bague, avec avantageusement dans la région proche de l'électrode 104 une bague additionnelle 126 incorporant un matériau susceptible de diffuser un agent stéroïde.

En ce qui concerne l'ensemble frontal 200, qui est mobile en rotation par rapport aux divers éléments 100 à 128 que l'on vient de décrire, cet ensemble comprend la bague support 204, qui est solidaire de la vis d'ancrage 202 et comprend une surface d'appui 206 tournée vers une surface homologue annulaire 130 du capot 116. Ces deux surfaces 130 et 206, toutes deux métalliques, forment une première interface 304 avec un effet de friction mutuelle.

La surface opposée 208 de la bague support 204 vient en appui contre un ressort annulaire 300, le ressort 300 et la bague 204 n'étant pas solidaires entre eux. Entre les deux éléments constitués par la surface 208 de la bague 204 et le ressort 300 se forme une deuxième interface 304 avec effet de friction mutuelle. Côté proximal, le ressort 300 vient en appui contre une surface 132 du capuchon 124.

Le ressort annulaire 300, qui se trouve donc ainsi axialement emprisonné entre la bague support 204 et le capuchon 124, exerce sur la bague support 204 un effort axial en direction du corps tubulaire 100, effort se traduisant par un effet de frottement aux interfaces 302 et 304.

Ce mécanisme de couplage frottant entre, d'une part, le corps tubulaire 100 et les différents éléments qui lui sont solidaires et, d'autre part, l'ensemble frontal 200 avec notamment la vis d'ancrage 202, permet de limiter le couple transmis par le corps tubulaire à la vis d'ancrage 202 et de réaliser ainsi l'effet limiteur de couple anti-carottage recherché.

On notera également qu'il reste toujours possible de réorienter axialement le corps tubulaire une fois la capsule ancrée dans la paroi, et ce sans exercer d'action de rotation sur la vis, donc sans risque de survissage pouvant entrainer un carottage.

Cette faculté peut se révéler intéressante dans le cas d'une capsule munie d'un récupérateur d'énergie tel que celui illustré Figure 2, comprenant une masse inertielle montée à l'extrémité d'une lame flexible : avec cette configuration, la récupération de l'énergie peut varier en fonction de l'orientation dans l'espace de la direction de flexion de la lame par rapport à la paroi cardiaque, de sorte qu'il peut être avantageux de tenter d'optimiser la conversion d'énergie en faisant tourner le corps de la capsule jusqu'à trouver la position qui maximise cette conversion.

Si, par ailleurs, on souhaite explanter la capsule, un couple de rotation exercé en sens inverse sur le corps tubulaire permettra de transmettre le mouvement de dévissage à la vis hélicoïdale et donc de détacher progressivement la capsule de la paroi.

Pour le ressort 300, le choix d'un ressort ondulé présente l'avantage d'une très grande compacité, compatible avec les exigences de miniaturisation extrême des capsules *leadless.* La longueur du ressort, c'est-à-dire l'intervalle compris entre les surfaces en vis-à-vis 208 et 132, est typiquement de l'ordre de a = 1,6 mm, ce qui permet de réaliser un ensemble de longueur réduite, par exemple de longueur b = 3 mm (la partie saillante de la vis, correspondant à la longueur pénétrant dans la paroi, étant typiquement de c = 1,6 mm environ).

L'avantage du ressort ondulé est le gain de place axial qu'il permet, typiquement de 50 % par rapport à un ressort filaire hélicoïdal conventionnel. D'autres types de ressorts ou éléments fonctionnement analogues sont toutefois envisageables, tels que : entretoise en matériau souple, par exemple en silicone, ressort hélicoïdal de compression, lame ressort, bague plastique déformable, etc., l'important étant que cet élément puisse engendrer un frottement suffisant pour limiter le couple à la valeur choisie, tout en maintenant l'orientation angulaire du corps tubulaire 100 par rapport à l'ensemble frontal en l'absence de couple appliqué de l'extérieur au corps tubulaire.

### Deuxième mode de réalisation de l'invention

La Figure 6 illustre un deuxième mode de réalisation de l'invention, où le corps tubulaire 100 et l'ensemble frontal 200 sont mobiles non seulement en rotation autour de l'axe D, mais également en translation axiale : plus précisément, l'ensemble formé par le corps tubulaire 100 avec les éléments qu'il porte, notamment l'électrode 104, peut reculer en direction distale (c'est-à-dire vers la gauche avec les conventions de la figure) par rapport à l'ensemble frontal 200. Avant ce mouvement de translation axiale, les deux ensembles 100 et 200 sont solidaires en rotation, mais une fois la translation axiale effectuée, ils deviennent libres en rotation relative.

La translation axiale a lieu quand, au moment du vissage, la face frontale de l'électrode 104 vient en contact avec la surface du tissu dans lequel la vis 202 est en train de pénétrer. L'électrode 104 et donc le corps tubulaire 100 ne peuvent plus progresser, alors que, du fait de la rotation, la vis d'ancrage 200 continue à pénétrer dans le tissu. Il en résulte un recul progressif (flèche 134) en direction axiale du corps tubulaire 100 et de l'électrode 104 par rapport à l'ensemble frontal 200 et à la vis 20, ceci jusqu'à produire un débrayage de ces deux ensembles l'un par rapport à l'autre.

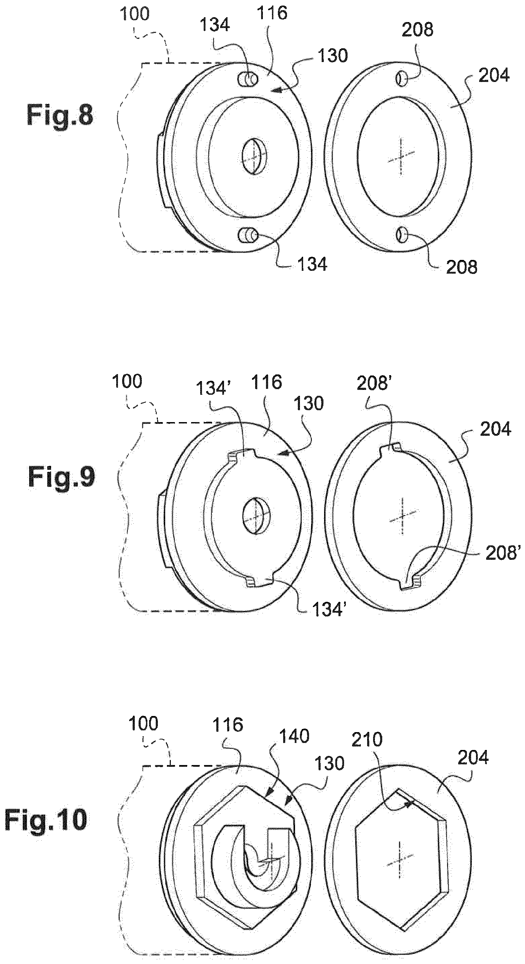

Pour ce faire, comme illustré Figure 8, la face 130 du capot 116 tournée vers la bague support est munie de pions 134 destinés à s'emboiter dans des alésages homologues 208 de la bague support 204. Par ailleurs, un élément élastique 306 (Figure 6), par exemple une bague silicone, est interposé entre le capuchon 124 et la bague support 204, de manière à exercer une force axiale d'un niveau prédéterminé entre la face 130 du capot 116 et la bague support 204.

De la sorte, la pénétration progressive de la vis 202 dans le tissu, qui a pour effet de repousser axialement l'électrode 104 et donc le corps tubulaire 100 et son capot de fermeture 116 (flèche 134) va avoir pour effet de progressivement sortir les pions 134 des alésages 208. Lorsque les pions seront totalement sortis des alésages, alors la bague support 204 sera découplée en rotation du capot 116, réalisant la fonction de limitation de couple recherchée.

Les Figures 9 et 10 illustrent des variantes de la Figure 8, dans laquelle les pions 134 et les alésages 208 sont remplacés par des excroissances 134' coopérant avec des encoches homologues 208' de la bague support 204 ou (Figure 10) par un profil non circulaire 140 (hexagonal dans l'exemple illustré) coopérant avec un profil conjugué 210 de la bague support 204.

La Figure 7 illustre une variante du mode de réalisation de la Figure 6.

Cette variante met en œuvre comme élément élastique des rondelles ondulées 308 interposées entre la bague support 204 et un épaulement 142 d'un élément annulaire 144 solidaire du capot 116.

La pénétration progressive de la vis 202 a pour effet de repousser l'électrode 104 et le corps tubulaire 100 en direction distale (vers la gauche avec les conventions de la figure), tandis que la vis 202 continuera de progresser dans le tissu, en direction proximale (vers la droite). Ce mouvement de translation relative a pour conséquence de comprimer les rondelles ondulées 308, jusqu'à produire un débrayage entre le capot de fermeture 116 et la bague support 204.

### Troisième mode de réalisation de l'invention

La Figure 11 illustre un troisième mode de réalisation de l'invention, dans lequel le corps tubulaire 100 et l'électrode 104, d'une part, et l'ensemble frontal 200 avec la vis d'ancrage 202, d'autre part, ne sont pas mobiles axialement, à la différence du mode de réalisation précédent.

Dans ce troisième mode de réalisation, l'élément élastique est constitué par la bague support 204 elle-même qui, comme on peut le voir sur la Figure 12 où elle a été illustrée isolément, est une bague annulaire fendue, avec une fente radiale 212. Cette fente radiale procure à la bague support 204 une élasticité radiale, à la manière d'un anneau élastique de type circlip.

La bague support 204 est par ailleurs munie à sa périphérie intérieure 214 de bossages 216 coopérant avec des creux homologues 142 prévus sur une surface extérieure 144 formant rampe d'un épaulement 146 du capot de fermeture 116.

Tant que le couple exercé sur le corps tubulaire 100, et donc le couple transmis au capot 116 et à la bague support 204 via les éléments conjugués 142, 216, est inférieur à un couple prédéterminé, la bague support 204 ne se déforme pas, ce qui permet la transmission de ce couple de rotation à la bague support 204 et donc à la vis 202. En revanche, lorsque le couple augmente au-delà de la valeur prescrite, la bague support fendue 204 se déforme radialement, de sorte que les bossages 216 quittent les évidements 142, réalisant ainsi la fonction recherchée de limitation de couple et de débrayage.

## Revendications

1. Une capsule autonome implantable, comprenant :
- un corps tubulaire (100) logeant un ensemble d'éléments fonctionnels de la capsule, et
- à une extrémité proximale (102) de la capsule, un ensemble frontal (200) comprenant un organe d'ancrage (202) pour l'ancrage de la capsule à une paroi d'un organe du patient,
**caractérisée en ce que** l'ensemble frontal (200) est, avec l'organe d'ancrage (200), mobile en rotation relative axiale par rapport au corps tubulaire (100),
et **en ce que** la capsule comprend en outre, entre l'ensemble frontal et le corps tubulaire :
- un organe de couplage débrayable agencé pour :
• autoriser ladite rotation relative lorsque le corps tubulaire reçoit une sollicitation externe en rotation qui lui est appliquée avec l'organe d'ancrage ancré dans la paroi de l'organe du patient, l'organe d'ancrage exerçant alors un couple de réaction supérieur à un couple seuil prédéterminé, et
• empêcher ladite rotation relative en l'absence de sollicitation externe en rotation appliquée au corps tubulaire.

2. La capsule de la revendication 1, dans laquelle l'organe de couplage débrayable comprend :
- au moins une interface de friction (302, 304) entre l'ensemble frontal et le corps tubulaire ; et
- un élément élastiquement déformable (300) agencé pour appliquer un effort de compression axiale entre une première face d'appui (132) solidaire en rotation du corps tubulaire (100) et une seconde face d'appui (208) solidaire en rotation de l'ensemble frontal (200).

3. La capsule de la revendication 2, dans laquelle, en l'absence de sollicitation externe en rotation appliquée au corps tubulaire, l'élément élastiquement déformable applique à l'endroit de l'interface de friction un effort supérieur au couple seuil prédéterminé.

4. La capsule de la revendication 2, dans laquelle l'organe de couplage débrayable comprend une bague support (204) solidaire en rotation de l'organe d'ancrage (202) et venant en appui frottant contre une surface annulaire frontale (130) du corps tubulaire (100) située à l'extrémité proximale de celui-ci.

5. La capsule de la revendication 4, dans laquelle l'élément élastiquement déformable (300) applique l'effort de compression axiale contre la bague support en direction de la surface annulaire frontale (130).

6. La capsule de la revendication 4, dans laquelle la bague support comprend deux faces (206, 208), comprenant :
- une première face (206) tournée vers la surface annulaire frontale (130), avec un degré de liberté en rotation et un premier contact frottant à l'interface (304) entre la première face et la surface annulaire frontale ; et
- une seconde face (208) opposée tournée vers l'élément élastiquement déformable (300), avec un degré de liberté en rotation et un second contact frottant à l'interface (302) entre la seconde face et l'élément élastiquement déformable.

7. La capsule de la revendication 2, dans laquelle le corps tubulaire (100) et l'ensemble frontal (200) ne sont pas mobiles entre eux en translation axiale.

8. La capsule de la revendication 1, dans laquelle l'organe de couplage débrayable comprend :
- une bague support (204) solidaire en rotation de l'organe d'ancrage (202) ;
- une surface frontale (116) du corps tubulaire (100), située à l'extrémité proximale de celui-ci en vis-à-vis de la bague support ; et
- des formes conjuguées (134, 208 ; 134', 208' ; 140, 210 ; 144, 214) prévues respectivement sur la bague support (204) et sur la surface frontale (116), les formes conjuguées permettant un emboitement mutuel du corps tubulaire (100) avec l'ensemble frontal (200) pour permettre un entrainement en rotation de la bague support (204), et corrélativement de l'organe d'ancrage (202) solidaire de celle-ci, par l'organe d'emboitement de la surface frontale sous l'effet de la sollicitation externe en rotation appliquée au corps tubulaire.

9. La capsule de la revendication 8, dans laquelle le corps tubulaire (100) et l'ensemble frontal (200) sont mobiles entre eux en translation axiale, et le corps tubulaire porte à son extrémité proximale un élément (104) destiné à venir en appui contre la paroi de l'organe du patient, cet élément (104) étant apte à exercer durant la pénétration de l'organe d'ancrage (202) un effort de réaction axial (136) propre à déboiter axialement les formes conjuguées respectives (134, 208 ; 134', 208'; 140, 210) de la bague support (204) et de la surface frontale (116).

10. La capsule de la revendication 9, dans laquelle l'organe de couplage débrayable comprend en outre un élément élastiquement déformable (306, 308) agencé pour solliciter axialement les formes conjuguées en rapprochement mutuel.

11. La capsule de la revendication 10, dans laquelle l'élément élastiquement déformable (306, 308) est agencé pour subir, sous l'effet dudit effort de réaction axial (136), une déformation d'un état non déformé à un état de déformation maximale correspondant audit couple seuil prédéterminé.

12. La capsule de la revendication 2 ou de la revendication 10, dans laquelle l'élément élastiquement déformable est un élément du groupe constitué par : bague ondulée (308), ressort de compression, lame ressort, bague plastique déformable (306) et entretoise en matériau souple.

13. La capsule de la revendication 8, dans laquelle le corps tubulaire (100) et l'ensemble frontal (200) ne sont pas mobiles entre eux en translation axiale, et dans laquelle l'organe de couplage débrayable comprend un élément élastiquement déformable (204, 212) agencé pour subir, sous l'effet dudit couple de réaction, une déformation entre :
- un état où l'élément élastiquement déformable exerce entre les formes conjuguées (144, 214) un effort de striction radiale assurant l'emboitement mutuel du corps tubulaire (100) avec l'ensemble frontal (200), et
- un état de déformation maximale propre à déboiter le corps tubulaire (100) et l'ensemble frontal (200), pour un couple de réaction correspondant audit couple seuil prédéterminé.

## Patentansprüche

1. Implantierbare autonome Kapsel, umfassend:
- einen rohrförmigen Körper (100), der eine Anordnung aus Funktionselementen der Kapsel aufnimmt, und
- an einem proximalen Ende (102) der Kapsel, eine frontale Anordnung (200), umfassend ein Verankerungsorgan (202) für die Verankerung der Kapsel an einer Wand eines Organs des Patienten,
**dadurch gekennzeichnet, dass** die frontale Anordnung (200), mit dem Verankerungsorgan (202) gegenüber dem rohrförmigen Körper (100) in axialer relativer Drehung beweglich ist,
und dadurch, dass die Kapsel ferner, zwischen der frontalen Anordnung und dem rohrförmigen Körper, umfasst:
- ein auskuppelbares Kupplungsorgan, das eingerichtet ist, um:
• die relative Drehung freizugeben, wenn der rohrförmige Körper eine externe Drehbeaufschlagung aufnimmt, die mit dem Verankerungsorgan, das in der Wand des Organs des Patienten verankert ist, auf ihn aufgebracht wird, wobei das Verankerungsorgan dann ein Reaktionsmoment ausübt, das größer ist als ein vorher bestimmtes Schwellendrehmoment, und
• die relative Drehung bei Fehlen einer auf den rohrförmigen Körper aufgebrachten externen Drehbeaufschlagung zu verhindern.

2. Kapsel nach Anspruch 1, wobei das auskuppelbare Kupplungsorgan umfasst:
- wenigstens eine Reibungsschnittstelle (302, 304) zwischen der frontalen Anordnung und dem rohrförmigen Körper; und
- ein elastisch verformbares Element (300), das eingerichtet ist, um eine axiale Druckkraft zwischen einer ersten Auflagefläche (132), die mit dem rohrförmigen Körper (100) drehfest verbunden ist, und einer zweiten Auflagefläche (208), die mit der frontalen Anordnung (200) drehfest verbunden ist, aufzubringen.

3. Kapsel nach Anspruch 2, wobei, bei Fehlen einer auf den rohrförmigen Körper aufgebrachten externen Drehbeaufschlagung, das elastisch verformbare Element (300) auf die Stelle der Reibungsschnittstelle eine Kraft aufbringt, die größer ist als das vorher bestimmte Schwellendrehmoment.

4. Kapsel nach Anspruch 2, wobei das auskuppelbare Kupplungsorgan einen Stützring (204) umfasst, der mit dem Verankerungsorgan (202) drehfest verbunden ist und gegen eine frontale ringförmige Fläche (130) des rohrförmigen Körpers (100), die sich an dem proximalen Ende von diesem befindet, in Reibauflage kommt.

5. Kapsel nach Anspruch 4, wobei das elastisch verformbare Element (300) die axiale Druckkraft gegen den Stützring in Richtung der frontalen ringförmigen Fläche (130) aufbringt.

6. Kapsel nach Anspruch 4, wobei der Stützring zwei Flächen (206, 208) umfasst, umfassend:
- eine erste Fläche (206), die zu der frontalen ringförmigen Fläche (130) hin weist, mit einem Drehfreiheitsgrad und einem ersten Reibkontakt an der Schnittstelle (304) zwischen der ersten Fläche und der frontalen ringförmigen Fläche; und
- eine gegenüberliegende zweite Fläche (208), die zu dem elastisch verformbaren Element (300) hin weist, mit einem Drehfreiheitsgrad und einem zweiten Reibkontakt an der Schnittstelle (302) zwischen der zweiten Fläche und dem elastisch verformbaren Element.

7. Kapsel nach Anspruch 2, wobei der rohrförmige Körper (100) und die frontale Anordnung (200) nicht in axialer Translation zueinander beweglich sind.

8. Kapsel nach Anspruch 1, wobei das auskuppelbare Kupplungsorgan umfasst:
- einen Stützring (204), der mit dem Verankerungsorgan (202) drehfest verbunden ist;
- eine frontale Fläche (116) des rohrförmigen Körpers (100), die sich an dem proximalen Ende von diesem dem Stützring gegenüber befindet; und
- angepasste Formen (134, 208; 134', 208'; 140, 210; 144, 214), die jeweils an dem Stützring (204) und an der frontalen Fläche (116) vorgesehen sind, wobei die angepassten Formen ein Ineinandergreifen des rohrförmigen Körpers (100) mit der frontalen Anordnung (200) gestatten, um ein Antreiben des Stützrings (204) zur Drehung, und dementsprechend des mit diesem drehfest verbundenen Verankerungsorgans (202), zu gestatten, durch das Eingreiforgan der frontalen Fläche unter der Einwirkung der auf den rohrförmigen Körper aufgebrachten externen Drehbeaufschlagung.

9. Kapsel nach Anspruch 8, wobei der rohrförmige Körper (100) und die frontale Anordnung (200) in axialer Translation zueinander beweglich sind und der rohrförmige Körper an seinem proximalen Ende ein Element (104) trägt, das dazu bestimmt ist, gegen die Wand des Organs des Patienten in Auflage zu kommen, wobei dieses Element (104) imstande ist, während des Eindringens des Verankerungsorgans (202) eine axiale Reaktionskraft (136) auszuüben, die imstande ist, die jeweiligen angepassten Formen (134, 208; 134', 208'; 140, 210) des Stützrings (204) und der frontalen Fläche (116) axial außer Eingriff zu bringen.

10. Kapsel nach Anspruch 9, wobei das auskuppelbare Kupplungsorgan ferner ein elastisch verformbares Element (306, 308) umfasst, das eingerichtet ist, um die angepassten Formen axial zur gegenseitigen Annäherung zu beaufschlagen.

11. Kapsel nach Anspruch 10, wobei das elastisch verformbare Element (306, 308) dazu eingerichtet ist, unter der Einwirkung der axialen Reaktionskraft (136), eine Verformung aus einem nicht verformten Zustand in einen maximalen Verformungszustand zu erfahren, der dem vorher bestimmten Schwellendrehmoment entspricht.

12. Kapsel nach Anspruch 2 oder Anspruch 10, wobei das elastisch verformbare Element ein Element der Gruppe ist, bestehend aus: Wellring (308), Druckfeder, Federzunge, plastisch verformbarer Ring (306) und Distanzring aus flexiblem Material.

13. Kapsel nach Anspruch 8, wobei der rohrförmige Körper (100) und die frontale Anordnung (200) nicht in axialer Translation zueinander beweglich sind, und wobei das auskuppelbare Kupplungsorgan ein elastisch verformbares Element (204, 212) umfasst, das eingerichtet ist, um, unter der Einwirkung des Reaktionsdrehmoments, eine Verformung zu erfahren zwischen:
- einem Zustand, wo das elastisch verformbare Element zwischen den angepassten Formen (144, 214) eine radiale Striktionskraft ausübt, die das Ineinandergreifen des rohrförmigen Körpers (100) mit der frontalen Anordnung (200) bewirkt, und
- einem Zustand der maximalen Verformung, die imstande ist, den rohrförmigen Körper (100) und die frontale Anordnung (200) außer Eingriff zu bringen, bei einem Reaktionsdrehmoment, das dem vorher bestimmten Schwellendrehmoment entspricht.

## Claims

1. An implantable autonomous capsule, comprising:
- a tubular body (100) housing a set of functional elements of the capsule, and
- at a proximal end (102) of the capsule, a front-end unit (200) comprising an anchoring member (202) for anchoring the capsule to a wall of a patient's organ,
**characterized in that** the front-end unit (200) is, along with the anchoring member (200), mobile in relative axial rotation with respect to the tubular body (100),
and **in that** the capsule further comprises, between the front-end unit and the tubular body:
- a disengageable coupling member adapted to:
• enable said relative rotation when the tubular body receives an external rotational stress that is applied thereto with the anchoring member anchored into the wall of the patient's organ, the anchoring member then exerting a reaction torque higher than a predetermined threshold torque, and
• prevent said relative rotation in the absence of external rotational stress applied to the tubular body.

2. The capsule of claim 1, wherein the disengageable coupling member comprises:
- at least one friction interface (302, 304) between the front-end unit and the tubular body; and
- an elastically deformable element (300) adapted to apply an axial compression force between a first bearing face (132), rotationally integral with the tubular body (100), and a second bearing face (208), rotationally integral with the front-end unit (200).

3. The capsule of claim 2, wherein, in the absence of external rotational stress applied to the tubular body, the elastically deformable element applies, at the friction interface, a force higher than the predetermined threshold torque.

4. The capsule of claim 2, wherein the disengageable coupling member comprises a support ring (204) rotationally integral with the anchoring member (202) and coming into frictional bearing against a front annular surface (130) of the tubular body (100), located at the proximal end of the latter.

5. The capsule of claim 4, wherein the elastically deformable element (300) applies the axial compression force against the support ring towards the front annular surface (130).

6. The capsule of claim 4, wherein the support ring comprises two faces (206, 208), comprising:
- a first face (206) directed towards the front annular surface (130), with a rotational degree of freedom and a first frictional contact at the interface (304) between the first face and the front annular surface; and
- a second, opposite face (208) directed towards the elastically deformable element (300), with a rotational degree of freedom and a second frictional contact at the interface (302) between the second face and the elastically deformable element.

7. The capsule of claim 2, wherein the tubular body (100) and the front-end unit (200) are not mobile in axial translation relative to each other.

8. The capsule of claim 1, wherein the disengageable coupling member comprises:
- a support ring (204) rotationally integral with the anchoring member (202);
- a front surface (116) of the tubular body (100), located at the proximal end of the latter, opposite the support ring; and
- conjugated shapes (134, 208 ; 134', 208'; 140, 210 ; 144, 214) respectively provided on the support ring (204) and on the front surface (116), the conjugated shapes allowing a mutual fitting of the tubular body (100) with the front-end unit (200) to allow the support ring (204), and correlatively the anchoring member (202) integral with the latter, to be rotated by the fitting member of the front surface under the effect of the external rotational stress applied to the tubular body.

9. The capsule of claim 8, wherein the tubular body (100) and the front-end unit (200) are mobile in axial translation relative to each other, and the tubular body can carry, at its proximal end, an element (104) intended to bear against the wall of the patient's organ, said element (104) being adapted, during the penetration of the anchoring member (202), to exert an axial reaction force (136) capable of axially disconnecting the respective conjugated shapes (134, 208 ; 134', 208' ; 140, 210 ; 144, 214) of the support ring (204) and of the front surface (116).

10. The capsule of claim 9, wherein the disengageable coupling member further comprises an elastically deformable element (306, 308) adapted to axially urge the conjugated shapes towards each other.

11. The capsule of claim 10, wherein the elastically deformable element (306, 308) is adapted, under the effect of said axial reaction force (136), to undergo a deformation from a non-deformed state to a maximum deformation state corresponding to said predetermined threshold torque.

12. The capsule of claim 2 or claim 10, wherein the elastically deformable element is an element of the group consisting in: corrugated ring (308), compression spring, leaf spring, deformable plastic ring (306), and spacer made from a flexible material.

13. The capsule of claim 8, wherein the tubular body (100) and the front-end unit (200) are not mobile in axial translation relative to each other, and the disengageable coupling member comprises an elastically deformable element (204, 212) adapted, under the effect of said reaction torque, to undergo a deformation between:
- a state in which the elastically deformable element exerts between the conjugated shapes (144, 214) a radial constriction force causing the mutual fitting of the tubular body (100) with the front-end unit (200), and
- a state of maximal deformation, for disconnecting the tubular body (100) and the front-end unit (200), under a reaction torque corresponding to said predetermined threshold torque.
